# EUROPEAN PATENT APPLICATION

(11) **EP 4 613 769 A1**
(43) Date of publication of application: **10.09.2025**
(21) Application number: 23885045.7
(22) Date of filing: 02.11.2023
(51) Int. Cl.: C07K 16/28, C07K 14/54, C07K 14/715, A61P 35/00, A61K 39/395

(54) **FUSION PROTEIN AND USE THEREOF**

(30) Priority: 02.11.2022 CN 202211364321
(71) Applicant: Beijing Changping Laboratory, Changping District Beijing 102206 (CN)
(72) Inventor: REN, Zhenhua, Beijing 102206 (CN); FU, Yangxin, Beijing 102206 (CN)
(74) Representative: Nederlandsch Octrooibureau
(86) International application number: PCT/CN2023/129321
(87) International publication number: WO 2024/094119

(57) **Abstract**

The present invention relates to a fusion protein comprising an antibody against T cell surface molecule, interleukin-2 and a Fc region of an immunoglobulin. The fusion protein is a heterodimer or homodimer. The present invention also relates to use of the fusion protein in the manufacture of an anti-tumor drug.

## Description

### TECHNICAL FIELD

The present invention relates to the field of biomedical technology. Specifically, the present invention relates to a fusion protein comprising a T cell antibody and interleukin-2, and use thereof.

### BACKGROUND

Cytokines play an important role in cancer immunotherapy. However, the role of natural cytokines in cancer therapy is often limited due to their extremely short half-life, limited tumour targeting and significant side effects [1]. IL-2 is currently approved for the treatment of patients with renal cancer and melanoma, and is also the first cytokine approved for the treatment of tumors [2-4]. More than one-third of patients with renal cell carcinoma (RCC) are diagnosed at an advanced stage, with an estimated 5-year survival rate of about 12% [5]. Although several target-based therapies have been approved for delaying the cancer progression of metastatic RCC (mRCC) over the past decades, the current therapies still fail to induce a complete response (CR) in most patients [6]. Due to the well-known immunogenicity of RCC, the only therapy known to induce CR in mRCC with a persistent efficacy is IL-2 immunotherapy [7]. However, CR can only be achieved in 7-9% of patients [4]. IL-2 is known to cause significant short-term toxicity which causes hospitalisation for all patients treated with IL-2 for observation [8, 9]. Therefore, how to improve the therapeutic efficacy of IL-2 is a major challenge in cancer therapy. There are several major problems in the current IL-2 therapies: 1) limited tumor tissue targeting, especially tumor-specific T cells; 2) severe side effects caused by the lymphatic and endothelial tissue targeting; 3) preferential activation of Tregs and limitation of the anti-tumor effect mediated by CTLs; 4) short half-life (<30 minutes) due to its small size and lymphatic tissue targeting.

Tumor tissues with higher immunity typically harbor a large number of T cells, but their dysfunction limits their ability to control tumors. PD1+ and TIM3+ TILs are considered to be terminally differentiated and dysfunctional TILs [10]. Anti-PD1/PD-L1 therapy may remove the brakes on T cell response and partially restore their functions, but can only achieve a complete reaction in a minority of patients [11]. It is observed that cytokines play an important role in the treatment with an antibody against PD1[12]. This raises the possibility that targeting TILs with cytokines may overcome the resistance to the antibody against PD1. Most studies have focused on targeting cytokines to the tumor cells [13-15]. This will increase the retention of cytokines in tumor tissues, but the accessibility of effector T cells to these cytokines may remain limited. At present, it is unclear whether the tumor cells or stromal cells are the ideal targets for the T cell- associated cytokines, as most tumor cells are not in contact with T cells, and the endocytosis of cytokines by tumor cells may reduce the accessibility of the TILs to cytokines. However, due to the high expression of cytokine receptors on Treg cells, it is difficult to deliver sufficient cytokines to the CD8+ T cells in tumor while avoiding the systemic toxicity.

### SUMMARY OF THE INVENTION

A new fusion protein is designed by linking a cytokine (preferably a low-affinity cytokine) to an antibody (such as an anti-TIM3 antibody/an anti-LAG3 antibody, etc.) to target tumor-specific CD8+ T cells within the tumor rather than Tregs. The binding of the new fusion protein to peripheral CD8+ T cells and Treg cells is low, which greatly reduces the peripheral depletion and toxic side effects. The treatment with the new fusion protein will primarily act on CD8+T cells in tumor rather than Treg cells. For example, said CD8+ T cells are the ones with high expression of PD1/TIM3, and the new fusion protein can reactivate tumor-specific PD1+TIM3+CD8+ TILs and generate a long-term memory response to prevent recurrence.

Specifically, the present invention firstly relates to a fusion protein, which is a heterodimer comprising:
(1) a first monomer, which is represented as I-F, F-I, A-I-F, A-F-I, I-A-F, I-F-A, F-I-A or F-A-I from the N-terminus to the C-terminus; and
(2) a second monomer, which is represented as A-F, F-A, A-I-F, A-F-I, I-A-F, I-F-A, F-I-A or F-A-I from the N-terminus to the C-terminus;
wherein, the first monomer and the second monomer are different;
wherein, I represents interleukin-2 (IL2), F represents a Fc region of an immunoglobulin, and A represents an antibody against T cell surface molecule,
the antibody against T cell surface molecule is in the form of a Fab or a scFv;
the first monomer and the second monomer are linked through the dimerization of the Fc single chains to form the heterodimer.

In some embodiments, the heterodimer comprises a combination of a first monomer and a second monomer as follows:
In some embodiments, the heterodimer comprises
(1) a first monomer, which is represented as I-F from the N-terminus to the C-terminus; and
(2) a second monomer, which is represented as A-F from the N-terminus to the C-terminus.

In some embodiments, the heterodimer comprises:
(1) a first monomer, which is represented as I-F from the N-terminus to the C-terminus; and
(2) a second monomer, which is represented as F-A from the N-terminus to the C-terminus.

In some embodiments, the heterodimer comprises:
(1) a first monomer, which is represented as I-F from the N-terminus to the C-terminus; and
(2) a second monomer, which is represented as A-I-F from the N-terminus to the C-terminus.

In some embodiments, the heterodimer comprises:
(1) a first monomer, which is represented as I-F from the N-terminus to the C-terminus; and
(2) a second monomer, which is represented as I-A-F from the N-terminus to the C-terminus.

In some embodiments, the heterodimer comprises:
(1) a first monomer, which is represented as I-F from the N-terminus to the C-terminus; and
(2) a second monomer, which is represented as F-I-A from the N-terminus to the C-terminus.

In some embodiments, the heterodimer comprises:
(1) a first monomer, which is represented as I-F from the N-terminus to the C-terminus; and
(2) a second monomer, which is represented as F-A-I from the N-terminus to the C-terminus.

In some embodiments, the heterodimer comprises:
(1) a first monomer, which is represented as F-I from the N-terminus to the C-terminus; and
(2) a second monomer, which is represented as A-F from the N-terminus to the C-terminus.

In some embodiments, the heterodimer comprises:
(1) a first monomer, which is represented as F-I from the N-terminus to the C-terminus; and
(2) a second monomer, which is represented as F-A from the N-terminus to the C-terminus.

In some embodiments, the heterodimer comprises:
(1) a first monomer, which is represented as F-I from the N-terminus to the C-terminus; and
(2) a second monomer, which is represented as A-I-F from the N-terminus to the C-terminus.

In some embodiments, the heterodimer comprises:
(1) a first monomer, which is represented as F-I from the N-terminus to the C-terminus; and
(2) a second monomer, which is represented as I-A-F from the N-terminus to the C-terminus.

In some embodiments, the heterodimer comprises:
(1) a first monomer, which is represented as F-I from the N-terminus to the C-terminus; and
(2) a second monomer, which is represented as F-I-A from the N-terminus to the C-terminus.

In some embodiments, the heterodimer comprises:
(1) a first monomer, which is represented as F-I from the N-terminus to the C-terminus; and
(2) a second monomer, which is represented as F-A-I from the N-terminus to the C-terminus.

In some embodiments, the heterodimer comprises:
(1) a first monomer, which is represented as A-I-F from the N-terminus to the C-terminus; and
(2) a second monomer, which is represented as A-F from the N-terminus to the C-terminus.

In some embodiments, the heterodimer comprises:
(1) a first monomer, which is represented as A-I-F from the N-terminus to the C-terminus; and
(2) a second monomer, which is represented as F-A from the N-terminus to the C-terminus.

In some embodiments, the heterodimer comprises:
(1) a first monomer, which is represented as A-I-F from the N-terminus to the C-terminus; and
(2) a second monomer, which is represented as I-A-F from the N-terminus to the C-terminus.

In some embodiments, the heterodimer comprises:
(1) a first monomer, which is represented as A-I-F from the N-terminus to the C-terminus; and
(2) a second monomer, which is represented as F-I-A from the N-terminus to the C-terminus.

In some embodiments, the heterodimer comprises:
(1) a first monomer, which is represented as A-I-F from the N-terminus to the C-terminus; and
(2) a second monomer, which is represented as F-A-I from the N-terminus to the C-terminus.

In some embodiments, the heterodimer comprises:
(1) a first monomer, which is represented as I-A-F from the N-terminus to the C-terminus; and
(2) a second monomer, which is represented as A-F from the N-terminus to the C-terminus.

In some embodiments, the heterodimer comprises:
(1) a first monomer, which is represented as I-A-F from the N-terminus to the C-terminus; and
(2) a second monomer, which is represented as F-A from the N-terminus to the C-terminus.

In some embodiments, the heterodimer comprises:
(1) a first monomer, which is represented as I-A-F from the N-terminus to the C-terminus; and
(2) a second monomer, which is represented as A-I-F from the N-terminus to the C-terminus.

In some embodiments, the heterodimer comprises:
(1) a first monomer, which is represented as I-A-F from the N-terminus to the C-terminus; and
(2) a second monomer, which is represented as F-I-A from the N-terminus to the C-terminus.

In some embodiments, the heterodimer comprises:
(1) a first monomer, which is represented as I-A-F from the N-terminus to the C-terminus; and
(2) a second monomer, which is represented as F-A-I from the N-terminus to the C-terminus.

In some embodiments, the heterodimer comprises:
(1) a first monomer, which is represented as F-I-A from the N-terminus to the C-terminus; and
(2) a second monomer, which is represented as A-F from the N-terminus to the C-terminus.

In some embodiments, the heterodimer comprises:
(1) a first monomer, which is represented as F-I-A from the N-terminus to the C-terminus; and
(2) a second monomer, which is represented as F-A from the N-terminus to the C-terminus.

In some embodiments, the heterodimer comprises:
(1) a first monomer, which is represented as F-I-A from the N-terminus to the C-terminus; and
(2) a second monomer, which is represented as A-I-F from the N-terminus to the C-terminus.

In some embodiments, the heterodimer comprises:
(1) a first monomer, which is represented as F-I-A from the N-terminus to the C-terminus; and
(2) a second monomer, which is represented as I-A-F from the N-terminus to the C-terminus.

In some embodiments, the heterodimer comprises:
(1) a first monomer, which is represented as F-I-A from the N-terminus to the C-terminus; and
(2) a second monomer, which is represented as F-A-I from the N-terminus to the C-terminus.

In some embodiments, the heterodimer comprises:
(1) a first monomer, which is represented as F-A-I from the N-terminus to the C-terminus; and
(2) a second monomer, which is represented as A-F from the N-terminus to the C-terminus.

In some embodiments, the heterodimer comprises:
(1) a first monomer, which is represented as F-A-I from the N-terminus to the C-terminus; and
(2) a second monomer, which is represented as F-A from the N-terminus to the C-terminus.

In some embodiments, the heterodimer comprises:
(1) a first monomer, which is represented as F-A-I from the N-terminus to the C-terminus; and
(2) a second monomer, which is represented as A-I-F from the N-terminus to the C-terminus.

In some embodiments, the heterodimer comprises:
(1) a first monomer, which is represented as F-A-I from the N-terminus to the C-terminus; and
(2) a second monomer, which is represented as I-A-F from the N-terminus to the C-terminus.

In some embodiments, the heterodimer comprises:
(1) a first monomer, which is represented as F-A-I from the N-terminus to the C-terminus; and
(2) a second monomer, which is represented as F-I-A from the N-terminus to the C-terminus.

Preferably, the Fc region of an immunoglobulin is a Fc region of natural immunoglobulin or a Fc region of an immunoglobulin without the ADCC effectcaused by a gene mutation; more preferably, the Fc region of an immunoglobulin is a Fc single chain of human IgG (preferably human IgG1, human IgG2, human IgG3 or human IgG4), and preferably, the amino acid sequence of the Fc region of an immunoglobulin without the ADCC effect caused by a gene mutation is as set forth in SEQ ID NO: 2.

In some embodiments, when the antibody against T cell surface molecule is in the form of a scFv, interleukin-2 is attached to the N-terminus of the scFv; or when the antibody against T cell surface molecule is in the form of a Fab, interleukin-2 is attached to the N-terminus of CH1 or CL of the Fab region, or interleukin-2 is attached to the C-terminus of CL of the Fab region.

The present invention also relates to a fusion protein which is a homodimer, wherein the monomer of the homodimer comprises I-F-A, A-F-I, I-A-F, A-I-F, F-I-A, or F-A-I from the N-terminus to the C-terminus; wherein, I represents interleukin-2, F represents a Fc region of an immunoglobulin, and A represents an antibody against T cell surface molecule, and wherein the antibody against T cell surface molecule is in the form of a Fab or a scFv, and the monomers are linked through the dimerization of the Fc single chains to form the homodimer.

In some embodiments, when the antibody against T cell surface molecule is in the form of a scFv, interleukin-2 is attached to the N-terminus or the C-terminus of the scFv; or when the antibody against T cell surface molecule is in the form of a Fab, interleukin-2 is attached to the N-terminus of CH1 or CL of the Fab region, or interleukin-2 is attached to the C-terminus of CL of the Fab region.

In some embodiments, interleukin-2, the antibody against T cell surface molecule, or the Fc region of an immunoglobulin is linked to each other directly or via a linker. Preferably, the linker is selected from (G4S)n, wherein n = 0, 1 or 2.

In some embodiments, the T cell surface molecule includes, but is not limited to, TIM3, LAG3, PD1, ICOS, GITR, CD27, CD137, 2B4, OX40, 4-1BB, TIGIT, CTLA-4, VISTA, and CD8; preferably, the T cell surface molecule is TIM3 or LAG3, and the antibody against T cell surface molecule is an anti-TIM3 antibody (aTIM3) or an anti-LAG3 antibody (aLAG3).

In some embodiments, the antibody against T cell surface molecule is a humanized antibody or a fully human antibody.

In some embodiments, the sequence of interleukin-2 is as set forth in SEQ ID NO: 1, or is a mutant of the sequence as set forth in SEQ ID NO: 1; preferably, the mutant comprises any one or any combinations of the following mutation sites: R38L, F42A, H16K, D20K, R38A, F42K, and K43E.

In some embodiments, the heterodimer comprises:
(1) a first monomer comprising sequentially from the N-terminus,
   i) a wild-type IL-2 protein with the sequence as set forth in SEQ ID NO. 1, or a mutant of the wild-type IL-2 protein; preferably, the mutant comprises any one or any combinations of the following mutation sites: R38L, F42A, H16K, D20K, R38A, F42K and K43E;
   ii) an essential linking structure (*e.g*., a (G4S)n linking sequence); preferably, the linking sequence is as set forth in SEQ ID NO.6; and
   iii) a Fc region of IgG with the sequence as set forth in SEQ ID NO. 2, or a No-ADCC-mutated Fc region of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5; and
(2) a second monomer selected from the group consisting of:
   i) a second monomer comprising:
      a) a Fab region of an anti-TIM3 antibody consisting of a polypeptide with the sequence as set forth in SEQ ID NO.7 (VL-CL of the anti-TIM3 antibody) and a polypeptide with the sequence as set forth in SEQ ID NO.8 (VH-CH1 of the anti-TIM3 antibody); or an anti-TIM3 single chain variable fragment (ScFv) with the sequence as set forth in SEQ ID NO. 9; and
      b) a Fc region of IgG with the sequence as set forth in SEQ ID NO. 2, or a No-ADCC-mutated Fc region of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5;
   ii) a second monomer comprising:
      a) a Fab region of an antibody consisting of a polypeptide with the sequence as set forth in SEQ ID NO. 13 (VL-CL of the anti-LAG3 antibody) and a polypeptide with the sequence as set forth in SEQ ID NO.14 (VH-CH1 of the anti-LAG3 antibody); or a polypeptide with the sequence as set forth in SEQ ID NO. 15 (anti-LAG3 scFv); and
      b) a Fc region of IgG with the sequence as set forth in SEQ ID NO. 2, or a No-ADCC-mutated Fc region of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5; or
   iii) a second monomer comprising:
      a) a Fab construct of an anti-LAG3 antibody consisting of a polypeptide with the sequence as set forth in SEQ ID NO. 16 (aLAG3 VH-CH1-Fc (knob)) and a polypeptide with the sequence as set forth in SEQ ID NO. 13 (aLAG3 VL-CL); or
      b) a polypeptide with the sequence as set forth in SEQ ID NO. 17 or SEQ ID NO. 22 (aLAG3 ScFv-Fc (knob)).

In some embodiments, the heterodimer comprises:
a first monomer, which is a polypeptide with the sequence as set forth in SEQ ID NO. 10 (laIL2-Fc); and
a second monomer, which is:
   (1) a second monomer consisting of a polypeptide with the sequence as set forth in SEQ ID NO. 11 (VH-CH1-Fc of the anti-TIM3 antibody) and a polypeptide with the sequence as set forth in SEQ ID NO. 7 (VL-CLof the light chain of the anti-TIM3 antibody); or
   (2) a polypeptide with the sequence as set forth in SEQ ID NO. 12 or SEQ ID NO. 22 (aTIM3 ScFv-Fc).

In some embodiments, the monomer of the homodimer comprises sequentially from the N-terminus,
(1) a wild-type IL-2 protein with the sequence as set forth in SEQ ID NO. 1, or a mutant of the wild-type IL-2 protein, wherein the mutant comprises any one or any combinations of the following mutation sites: R38L, F42A, H16K, D20K, R38A, F42K and K43E;
(2) an essential linking structure (*e.g*., a (G4S)n linking sequence), preferably, the linking sequence is as set forth in SEQ ID NO.6;
(3) a Fab region or ScFv of an anti-TIM3 antibody, wherein the anti-TIM3 antibody is a humanized antibody or a fully human antibody;
(4) a Fc region of an antibody, which is a fully human wild-type Fc region or a No-ADCC-mutated Fc region.

In some embodiments, the monomer of the homodimer is:
(1) a polypeptide as set forth in SEQ ID NO. 18 (TIM3-InFc-laIL2: aTIM3(ScFv)-InFc-laIL2), or
(2) a polypeptide as set forth in SEQ ID NO. 19 (laIL2-InFc-TIM3: laIL2-Fc-aTIM3(ScFv)), or
(3) a polypeptide as set forth in SEQ ID NO. 20 (TIM3-laIL2-InFc: aTIM3(ScFv)-Fc-laIL2), or
(4) a polypeptide as set forth in SEQ ID NO. 21 (laIL2-TIM3-InFc: laIL2-aTIM3(ScFv)-Fc).

In some embodiments, the present invention further relates to another fusion protein, wherein the fusion protein is a fusion protein of an anti-LAG3 antibody and IL2, and the fusion protein is a heterodimer comprising:
(1) a first monomer comprising sequentially from the N-terminus:
   1) a wild-type IL-2 protein wth the sequence as set forth in SEQ ID NO. 1, or a mutant of the wild-type IL-2 protein, wherein the mutant comprises any one or any combinations of the following mutation sites: R38L, F42A, D20K, R38A, F42K and K43E;
   2) an essential linking structure (*e.g*., a (G4S)n linking sequence), preferably, the linking sequence is as set forth in SEQ ID NO.6; and
   3) a Fc region of IgG with the sequence as set forth in SEQ ID NO. 2, or a No-ADCC-mutated Fc region of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5; and
(2) a second monomer comprising:
   i) a Fab region of an antibody consisting of a polypeptide with the sequence as set forth in SEQ ID NO. 13 (VL-CL of the light chain of the anti-LAG3 antibody) and a polypeptide with the sequence as set forth in SEQ ID NO.14 (VH-CH1 of the heavy chain of the anti-LAG3 antibody); or a polypeptide with the sequence as set forth in SEQ ID NO. 15 (the anti-LAG3 scFv); and
   ii) a Fc region of IgG with the sequence as set forth in SEQ ID NO. 2, or a No-ADCC-mutated Fc region of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5;

In some embodiments, the heterodimer comprises:
a first monomer, which is a polypeptide with the sequence as set forth in SEQ ID NO. 10 (laIL2-Fc); and
a second monomer, which is:
   (1) a second monomer consisting of a polypeptide with the sequence as set forth in SEQ ID NO. 16 (aLAG3 VH-CH1-Fc) and a polypeptide with the sequence as set forth in SEQ ID NO. 13 (VL-CL of the light chain of the anti-LAG3 antibody); or
   (2) a polypeptide with the sequence as set forth in SEQ ID NO. 17 (aLAG3 scFv-Fc).

The present invention further relates to a fusion protein that is a homodimer, wherein the monomer of the homodimer is:
a monomer consisting of one molecule of interleukin-2 (IL2), one molecule of Fc single chain and one molecule of anti-TIM3 antibody or Fab region of the anti-LAG3 antibody linked directly or via a linker; or,
a monomer consisting of one molecule of interleukin-2 (IL2), one molecule of Fc single chain and one molecule of anti-TIM3 antibody or anti-LAG3 single chain variable fragment (scFv) linked directly or via a linker.

In some embodiments, the monomer of the homodimer comprises sequentially from the N-terminus,
(1) a wild-type IL-2 protein with the sequence as set forth in SEQ ID NO. 1, or a mutant of the wild-type IL-2 protein, whrein the mutant comprises any one or any combinations of the following mutation sites: R38L, F42A, H16K, D20K, R38A, F42K and K43E;
(2) an essential linking structure (*e.g*., a (G4S)n linking sequence); preferably, the linking sequence is as set forth in SEQ ID NO.6;
(3) a Fab region or ScFv of an anti-LAG3 antibody, wherein the anti-LAG3 antibody is a humanized antibody or a fully human antibody; and
(4) a Fc region of an antibody, which is a fully human wild-type Fc region or a No-ADCC-mutated Fc region.

In some embodiments, the monomer of the homodimer is:
(1) a polypeptide as set forth in SEQ ID NO. 22 (LAG3-InFc-laIL2: aLAG3(ScFv)-InFc-laIL2), or
(2) a polypeptide as set forth in SEQ ID NO. 23 (laIL2-InFc-LAG3: laIL2-Fc-aLAG3(ScFv)), or
(3) a polypeptide as set forth in SEQ ID NO. 24 (LAG3-laIL2-InFc: aLAG3(ScFv)-Fc-laIL2), or
(4) a polypeptide as set forth in SEQ ID NO. 25 (laIL2-LAG3-InFc: laIL2-aLAG3(ScFv)-Fc).

The present invention further relates to use of the fusion protein as follows:
(1) in the manufacture of an anti-tumor drug;
(2) in the manufacture of an anti-tumor kit, wherein the kit further comprises an immune checkpoint inhibitor or the T cells used in an adoptive T cell transfer therapy;
(3) in the manufacture of an anti-tumor drug for overcomeing a tolerance to an immune checkpoint inhibitor; or
(4) in the manufacture of an anti-tumor drugfor overcoming a non-responsiveness to an adoptive T cell transfer therapy,

preferably, the immune checkpoint inhibitor is an anti-PD-L1 antibody, an anti-PD1 antibody, or an anti-CTLA4 antibody;
preferably, the T cell is an anti-tumor T cell; more preferably, the T cell is an anti-tumor CAR T cell or a structural analogue thereof.

In another aspect, the present invention further relates to a kit comprising the fusion protein of the present invention as described above, preferably further comprising an anti-tumor drug selected from one or more of: an anti-PD-L1 antibody, an anti-PD1 antibody, an anti-TIGIT antibody and a chemotherapeutic drug, preferably a chemotherapeutic drug such as a tyrosine kinase inhibitor, nitrogen mustard, adriamycin, a platinum-based drug or paclitaxel.

In yet another aspect, the present invention relates to an mRNA or a DNA encoding the first monomer or the second monomer of the fusion protein, or encoding the monomer of the fusion protein.

In specific embodiments, the tumor is selected from lung cancer, colorectal cancer, gastric cancer, melanoma, renal cancer, lymphoma such as B-cell lymphoma, prostate cancer, thyroid cancer, breast cancer, pancreatic cancer, liver cancer, cervical cancer, ovarian cancer or esophageal cancer.

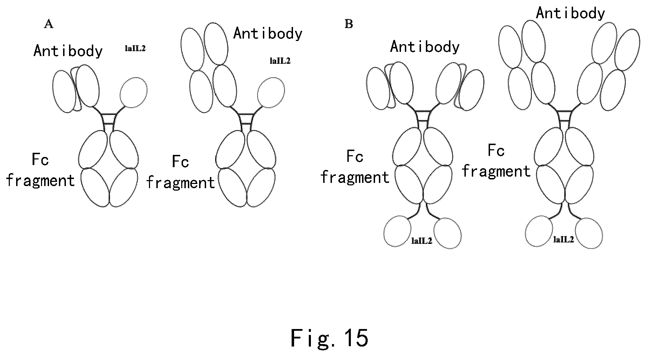

The structure of the exemplary fusion protein of the present invention is shown in Figure 15.

### BRIEF DESCRIPTION OF THE DRAWINGS

Figure 1. LAG3-laIL2 has a lower affinity with peripheral Tregs than LAG3-IL2.
Figure 2. TIM3-laIL2 has a lower affinity with peripheral Tregs than TIM3-IL2.
Figure 3. TIM3-laIL2 can bind to CD8 cells in tumor.
Figure 4. LAG3-laIL2 can bind to CD8 cells in tumor.
Figure 5. TIM3-laIL2 can effectively control the growth of MC38 tumor.
Figure 6. LAG3-laIL2 can effectively control the growth of MC38 tumor.
Figure 7. The anti-tumor effect of TIM3-laIL2 is significantly better than that of the combined treatment with Anti-TIM3 antibody and laIL2.
Figure 8. The anti-tumor effect of LAG3-laIL2 is significantly better than that of the combined treatment with Anti-LAG3 antibody and laIL2.
Figure 9. The anti-tumor effect of TIM3-laIL2 does not depend on CD4 T cells.
Figure 10. The anti-tumor effect of TIM3-laIL2 depends on CD8 T cells.
Figure 11. TIM3-laIL2 can synergize with the anti-PD-L1 antibody to control the tumor.
Figure 12. LAG3-laIL2 can synergize with the anti-PD-L1 antibody to control the tumor.
Figure 13. LAG3-InFc-laIL2 can effectively control the growth of MC38 tumor.
Figure 14. LAG3-InFc-laIL2 can effectively control the growth of CFPAC1 tumor.
Figure 15. Structural diagram of the exemplary fusion protein. Panel A shows the heterodimer forms; and panel B shows the homodimer forms.

### DETAILED DESCRIPTION OF THE INVENTION

### Experimental Materials

### 1. Strains and Plasmids

Strains: Top10 *E. coli* competent cell and DH5α *E. coli* competent cell (purchased from Beijing TransGen Biotech Co., Ltd.)
Plasmids:
   pEE6.4-IgGK-hIgG1 (purchased from Lonza), which comprises the signal peptide of mouse IgGκ and the sequence of the Fc region of human IgG1, and is used to express an antibody.
   pEE6.4-IgGκ-hIgG1-Fc-hole and pEE6.4-IgGκ-hIgG1-Fc-knob are used to express a heterodimeric protein.
   pEE6.4-TIM3 VH-CH1-Fc-knob and pEE6.4-TIM3 VL-CL are used to express the TIM3 antibody portion of a heterodimeric protein; and pEE6.4-laIL2-Fc-hole is used to express the laIL2 portion of a heterodimeric protein.

### 2. Experimental Animals

Wild-type C57BL/6 and BALB/c mice were purchased from Beijing Charles River Laboratory Animal Center (China), and NCG mice were purchased from GemPharmatech Biotechnology Co., Ltd. Unless otherwise specified, 8- to 10-week-old mice were used for all experiments. Mice were fed in a specific pathogen-free (SPF) barrier environment.

### 3. Cell Lines

MC38 (purchased from ATCC) is a C57 mouse-derived colorectal cancer cell line, and is cultured in DMEM complete medium (containing 10% inactivated fetal bovine serum, 2 mmol/l L-glutamine, 0.1 mmol/l non-essential amino acids, 100U penicillin and 100 µg/ml streptomycin).

A20 (purchased from ATCC) is a BALB/c mouse-derived B cell lymphoma cell line, and is cultured in RPMI1640 complete medium (containing 10% inactivated fetal bovine serum, 2mmol/L L-glutamine, 0.1mmol/L non-essential amino acids, 100U penicillin and 100µg/ml streptomycin).

FreeStyle^{™} 293F cell line (purchased from Invitrogen) is a suspension cell derived from HEK293 cell line, is cultured in SMM293-TII or CD OptiCHO^{™} culture medium and mainly used for transient transfection to express a fusion protein.

The sequence of IL2 gene containing the R38L and F42A mutations is as set forth in SEQ ID NO: 32, and represented as laIL2.

The proteins used in the experiments were designed by SnapGene software and synthesized by General Biol Co., Ltd.

**The followings are the proteins used in the Examples, wherein the anti-TIM3 antibody is in the form of a Fab, and the anti-LAG3 antibody is in the form of a scFv. Specifically:**
1. TIM3-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer consisting of aTIM3-VL-CL as set forth in SEQ ID NO. 26 and aTIM3-VH-CH1-InFc (hole) as set forth in SEQ ID NO. 27;
2. TIM3-IL2 consists of a first monomer and a second monomer as follows:
   (1) a polypeptide fused by wild-type IL2 and a Fc fragment, with the sequence as aet forth in SEQ ID NO. 31, and
   (2) a second monomer consisting of aTIM3-VL-CL as set forth in SEQ ID NO. 26 and aTIM3-VH-CH1-InFc (hole) as set forth in SEQ ID NO. 27;
3. LAG3-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer of aLAG3 as set forth in SEQ ID NO. 17;
4. LAG3-IL2 consists of a first monomer and a second monomer as follows:
   (1) a polypeptide fused by wild-type IL2 and a Fc fragment, with the sequence as set forth in SEQ ID NO. 31, and
   (2) a second monomer of aLAG3 as set forth in SEQ ID NO. 17;
5. PD1-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer of aPD1-ScFv-Fc (hole) as set forth in SEQ ID NO. 28;
6. hTIM3-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer of aTIM3 as set forth in SEQ ID NO. 12;
7. hLAG3-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer of aLAG3 as set forth in SEQ ID NO. 17;
8. hPD1-laIL2 consists of a first monomer and a second monomer as follows:
   (1) a polypeptide fused by IL2 containing the R38L and F42A mutations and a Fc fragment, with the sequence as set forth in SEQ ID NO. 10, and
   (2) a second monomer of ahPD1-ScFv-Fc(hole) as set forth in SEQ ID NO. 29;
9. aTIM3 antibody consists of aTIM3-VL-CL as set forth in SEQ ID NO. 26 and aTIM3-VH-CH1-Fc as set forth in SEQ ID NO. 30;
10. a PD-L1 antibody is a commercialized atezolizumab;
11. LAG3-InFc-laIL2 consists of the sequence as set forth in SEQ ID NO. 22.

### Tumor cells inoculation and treatment of mouse

### (1) Tumor cells inoculation and measurement:

Establishment of the tumor models: 1×10⁶ MC38 single cells were suspended in 100 µl PBS and inoculated subcutaneously into the back of a C57BL/6 mouse; 1.5× 10⁶ A20 single cells were suspended in 100 µl PBS and inoculated subcutaneously into the back of a BALB/c mouse; and 1×10⁶ CFPAC1 single cells were suspended in 100 µl PBS and inoculated subcutaneously into the back of a NCG mouse. For a re-challenge experiment of the same tumor cell in the mouse with regressed tumor, the number of tumor cells inoculated subcutaneously on the other side of the back of the mouse was 5 times that of the initial tumor modelling.

Tumor size was measured twice a week, and the long diameter (a), the short diameter (b) and the height (c) of a tumor were measured using a vernier caliper. The tumor volume of mouse= a×b×c/2.

### (2) Treatment:

The antibodies or fusion proteins were injected intraperitoneally, and the specific administration doses are given in the Examples.

### Depletion of CD4+T cells and CD8+T Cells in mouse

200 µg of GK1.5 or TIB210 antibody (BioXCell) was injected intraperitoneally one day before the treatment with IL2 or the IL2 fusion protein to deplete CD4+T cells and CD8+T cells, and then injected once every 3 days. The number of injections was adjusted according to the treatment cycle. The efficiency of depletion was determined by flow cytometry.

### Example 1: TIM3-laIL2 and LAG3-laIL2 have extremely low peripheral binding, thereby avoiding the peripheral side effects

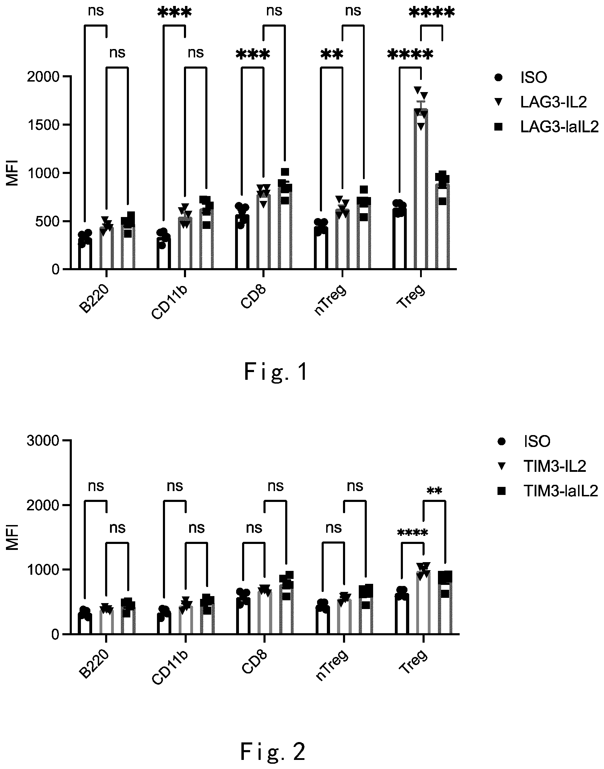

The binding ability of IL2 to IL2R α and IL2R β was reduced in order to reduce the toxicity of IL2. The binding ability of low-affinity IL2 (laIL2) fused with different antibodies to peripheral T cells and T cells in tumors was tested. 1µg/ml of different proteins were added to the single cell suspension of spleen for staining. As shown in **Figure 1****,** LAG3-laIL2 has a lower affinity than LAG3-IL2 for peripheral spleen Tregs. This can reduce the depletion of LAG3-laIL-2 by peripheral Treg cells. As shown in **Figure 2****,** TIM3-laIL2 has a lower affinity than TIM3-IL2 for peripheral spleen Tregs. This can reduce the depletion of TIM3-laIL2 by peripheral Treg cells.

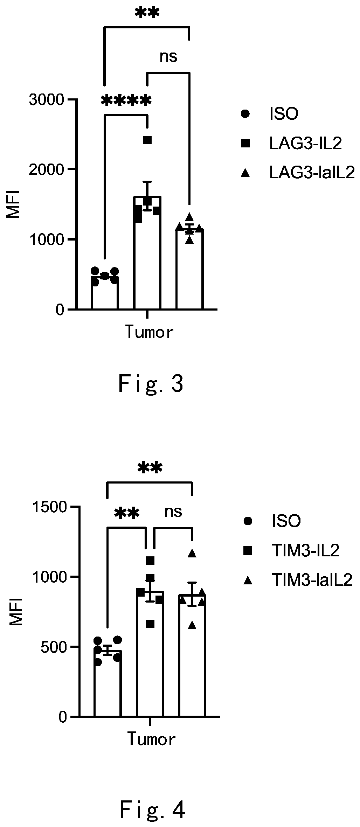

At the same time, the binding of LAG3-laIL2 and TIM3-laIL2 to T cells in tumors was also tested. 1µg/ml of different proteins were added to the single cell suspensions of tumors for staining. It can be seen that LAG3-laIL2 and TIM3-laIL2 can effectively bind to T cells in tumors **(****Figure 3 , 4).**

### Example 2: TIM3-laIL2 fusion protein and LAG3-laIL2 fusion protein have better anti-tumor activities

### 1. TIM3-laIL2 fusion protein can effectively control MC38 tumor.

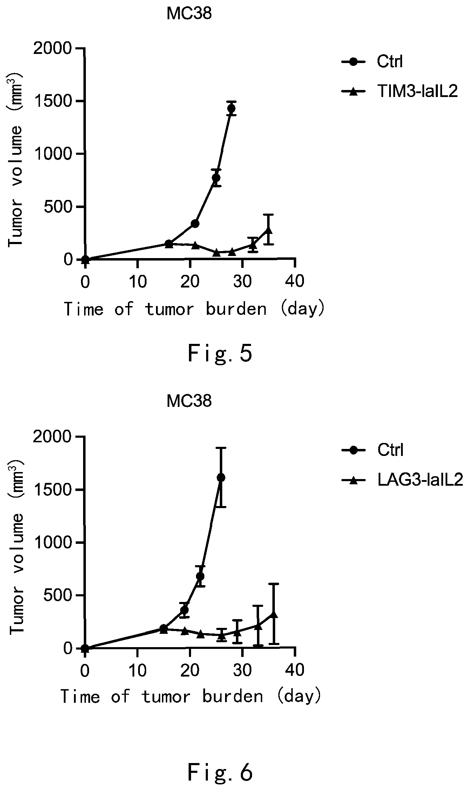

C57BL/6 mouse was inoculated subcutaneously with 1×10⁶ MC38 tumor cells, and treated on day 13 after tumor inoculation with the intraperitoneal injection of 30µg of TIM3-laIL2 antibody protein. Tumor size was measured twice a week. The results showed that TIM3-laIL2 fusion protein has a good therapeutic effect **(****Figure 5****).**

### 2. LAG3-laIL2 fusion protein can effectively control MC38 tumor.

C57BL/6 mouse was inoculated subcutaneously with 1×10⁶ MC38 tumor cells, and treated on day 13 after tumor inoculation with the intraperitoneal injection of 30µg of LAG3-laIL2 antibody protein. Tumor size was measured twice a week. The results showed that LAG3-laIL2 fusion protein has a good therapeutic effect **(****Figure 6****).**

### 3. TIM3-laIL2 fusion protein showed a significantly improved effect over the combined treatment in A20 B cell lymphoma.

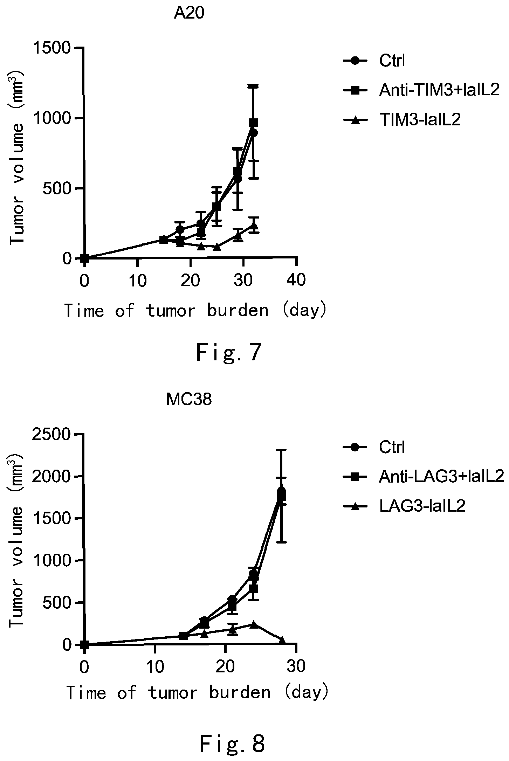

BALB/c mouse was inoculated subcutaneously with 1.5× 10⁶ A20 tumor cells, and treated on day D16 after tumor inoculation with the intraperitoneal injection of 15µg of aTIM3 antibody and 30µg of ahTIM3-laIL2, or 30µg of TIM3-laIL2 antibody protein, respectively. Tumor size was measured twice a week. The results showed that TIM3-laIL2 fusion protein has a better therapeutic effect compared to the combined treatment with aTIM3 antibody and laIL2 **(****Figure 7****).**

### 4. LAG3-InFc-laIL2 fusion protein showed a significantly improved effect over the treatment alone in MC38 colorectal cancer tumor.

C57BL/6 mouse was inoculated subcutaneously with 1×10⁶ MC38 tumor cells, and treated on day15 after tumor inoculation with the intraperitoneal injection of 15µg of aLAG3 antibody and 30µg of ahLAG3-laIL2 or 30µg of LAG3-laIL2 antibody protein, respectively. Tumor size was measured twice a week. The results showed that LAG3-laIL2 fusion protein has a better therapeutic effect compared to the combined treatment with aLAG3 antibody and laIL2 **(****Figure 8****).**

### Example 3. TIM3-laIL2 can activate CD8 T cells

### 1. The therapeutic effect of TIM3-laIL2 fusion protein does not depend on CD4 cells.

In order to determine which population of immune cells the treatment with TIM3-laIL2 antibody mainly depends on, depletion experiments of different cell populations were performed, respectively.

BALB/c mouse was inoculated subcutaneously with 1.5×10⁶ A20 tumor cells, and injected intraperitoneally with 30µg of TIM3-laIL2 fusion protein on day 15. 200µg of CD4 T cell-depleted antibody (clone number: GK1.5, purchased from BioXCell) was injected intraperitoneally one day before the start of treatment, once every 4 days for a total of 3 injections.

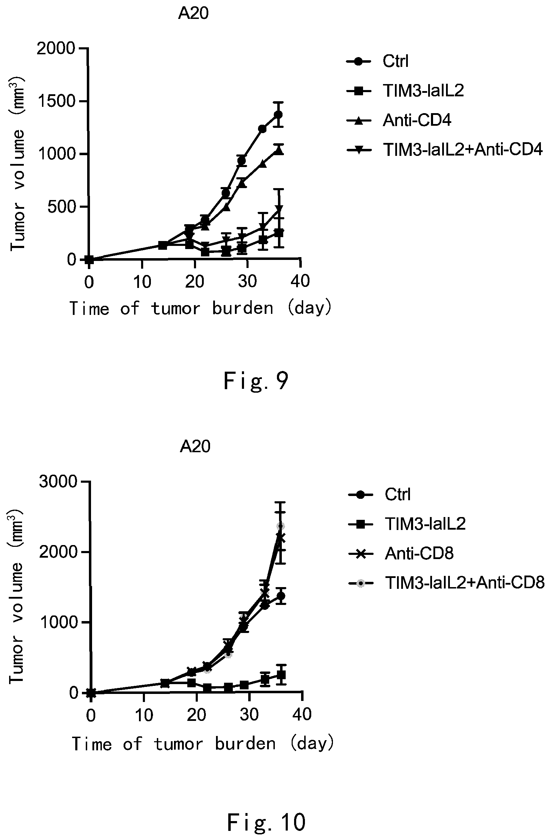

In the mouse tumor experiment, TIM3-laIL2 antibody still has a therapeutic effect after depleting CD4 cells, indicating that CD4 cells are not the main effector cells for the antibody to exert a therapeutic effect **(****Figure 9****).**

### 2. The therapeutic effect of TIM3-laIL2 depends on CD8 T cells.

- The role of CD8 T cells during the antibody treatment was further verified. BALB/c mouse was inoculated subcutaneously with 1.5×10⁶ A20 tumor cells, and treated on day 15 with the intraperitoneal injection of 30µg of TIM3-laIL2 protein. 200µg of CD8 T cell-depleted antibody (clone number: TIB210, purchased from BioXCell) was injected intraperitoneally one day before the start of treatment, once every 4 days for a total of 3 injections.

The depletion experiment of CD8 T cells showed that the therapeutic effect was significantly reduced after depleting CD8 T cells. This indicates that the therapeutic effect of TIM3-laIL2 antibody mainly depends on CD8 T cells (Figure 10).

### Example 4: TIM3-laIL2 and LAG3-laIL2 can overcome the non-responsiveness to the anti-PD-L1 antibody treatment

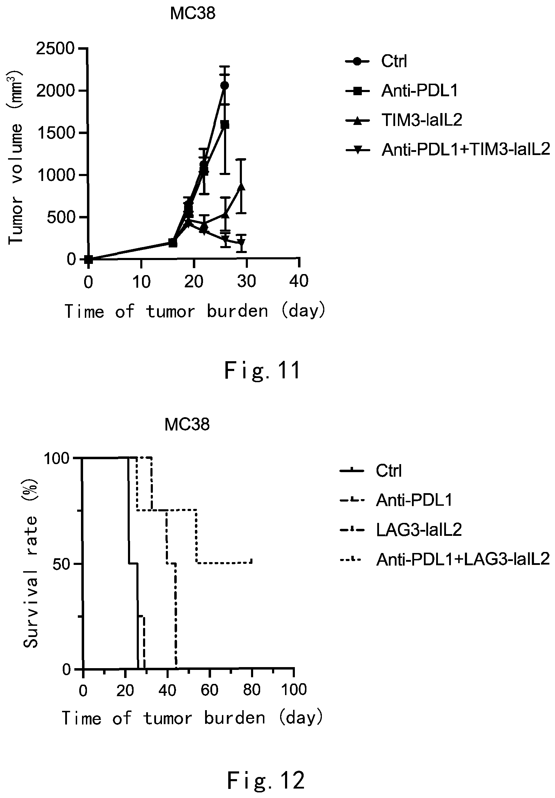

The treatment with TIM3-laIL2 or LAG3-laIL2 antibody alone has a good clearance effect in a MC38 tumor-bearing mouse with a tumor size below 150 mm³ **(****Figure 7****, 8).** However, the antibody treatment alone can only control the growth of tumor but not completely eliminate it when the tumor is larger **(****Figure 11****).** In order to further improve the therapeutic effect, the fusion protein and anti-immune checkpoint antibody were combined to observe whether the therapeutic effect can be improved or not.

Specific experimental protocol: C57BL/6 mouse was inoculated subcutaneously with 1×10⁶ MC38 tumor cells, and treated on day 19 after tumor inoculation with the intraperitoneal injection of 100µg of the anti-PD-L1 antibody or/and 30µg of TIM3-laIL2 antibody protein, respectively. Tumor size was measured twice a week. The results showed that the combined treatment has a better therapeutic effect compared to the treatment with the anti-PD-L1 antibody or TIM3-laIL2 fusion protein alone **(****Figure 11****).**

C57BL/6 mouse was inoculated subcutaneously with 1×10⁶ MC38 tumor cells, and treated on day 19 after tumor inoculation with the intraperitoneal injection of 100µg of the anti-PD-L1 antibody or/and 30µg of LAG3-laIL2 antibody protein, respectively. Tumor size was measured twice a week. The results showed that the combined treatment has a better therapeutic effect compared to the treatment with the anti-PD-L1 antibody or LAG3-laIL2 fusion protein alone

### (Figure 12).

### Example 4: LAG3-InFc-laIL2 fusion protein has a better anti-tumor activity

### 1. LAG3-InFc-laIL-2 fusion protein showed a significantly improved effect over the combined treatment in the tumor model of MC38 colorectal cancer.

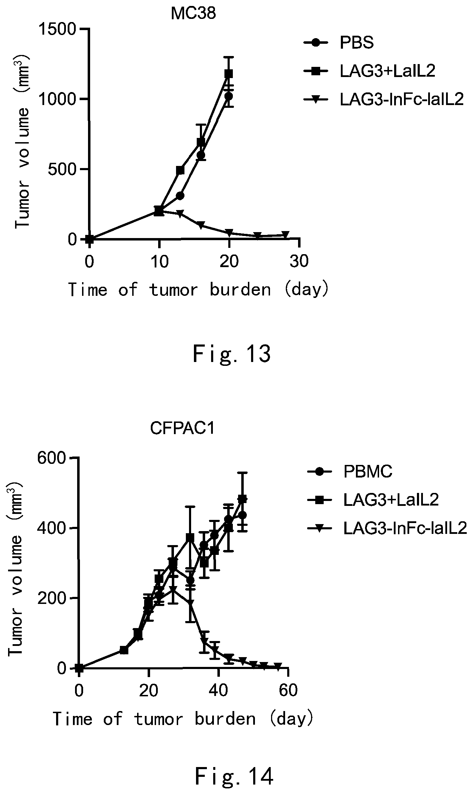

C57BL/6 mouse was inoculated subcutaneously with 1×10⁶ MC38 tumor cells, and treated on days 9 and 12 after tumor inoculation with the intraperitoneal injection of 15µg of aLAG3 antibody and 15µg of laIL2-Fc, or 30µg of LAG3-InFc-laIL2 antibody protein, respectively. Tumor size was measured twice a week. The results showed that LAG3-InFc-laIL2 fusion protein has a better therapeutic effect compared to the combined treatment with aLAG3 antibody and laIL2 (Figure 13).

2. TIM3-InFc-laIL2 fusion protein showed a significantly improved effect over the combined treatment in human CFPAC1 tumor model.

NCG mouse was inoculated subcutaneously with 1×10⁶ CFPAC1 tumor cells (purchased from ATCC), and was injected intravenously with 1×10⁶ PBMC on day13 after tumor inoculation. Treatment was performed on days 17 and 20 with the the intraperitoneal injection of 15µg of aLAG3 antibody and 15µg of laIL2, or 30µg of LAG3-InFc-laIL2 antibody protein, respectively. Tumor size was measured twice a week. The results showed that LAG3-InFc-laIL2 fusion protein has a better therapeutic effect compared to the combined antibody treatment (Figure 14).

The above examples are provided merely to assist those skilled in the art to understand the essence of the present invention, and are not provided to limit the protection scope of the present invention.

### References

1. Saxton, R.A., C.R. Glassman, and K.C. Garcia, Emerging principles of cytokine pharmacology and therapeutics. Nat Rev Drug Discov, 2022.
2. Tarhini, A.A. and S.S. Agarwala, Interleukin-2 for the treatment of melanoma. Curr Opin Investig Drugs, 2005. 6(12): p. 1234-9.
3. Hess, V., et al., Interleukin-2-based biochemotherapyfor patients with stage IV melanoma: long-term survivors outside a clinical trial setting. Oncology, 2007. 73(1-2): p. 33-40.
4. Hotte, S., et al., Interleukin-2 in the treatment of unresectable or metastatic renal cell cancer: a systematic review and practice guideline. Can Urol Assoc J, 2007. 1(1): p. 27-38.
5. Ged, Y., et al., The shifting treatment paradigm of metastatic renal cell carcinoma. Nat Rev Urol, 2022.
6. Brown, J.E. and S.N. Symeonides, Treatment Strategies in Metastatic Renal Cancer: Dose Titration in Clear Cell Renal Cell Carcinoma. Eur Urol, 2022. 82(3): p. 293-294.
7. Fishman, M., et al., Overall survival by clinical risk category for high dose interleukin-2 (HD IL-2) treated patients with metastatic renal cell cancer (mRCC): data from the PROCLAIM(SM) registry. J Immunother Cancer, 2019. 7(1): p. 84.
8. Acquavella, N., et al., Toxicity and activity of a twice daily high-dose bolus interleukin 2 regimen in patients with metastatic melanoma and metastatic renal cell cancer. J Immunother, 2008. 31(6): p. 569-76.
9. Alwan, L.M., et al., Comparison of acute toxicity and mortality after two different dosing regimens of high-dose interleukin-2 for patients with metastatic melanoma. Target Oncol, 2014. 9(1): p. 63-71.
10. Apetoh, L., et al., Consensus nomenclature for CD8(+) T cell phenotypes in cancer. Oncoimmunology, 2015. 4(4): p. e998538.
11. Vesely, M.D., T. Zhang, and L. Chen, Resistance Mechanisms to Anti-PD Cancer Immunotherapy. Annu Rev Immunol, 2022. 40: p. 45-74.
12. Ren, Z., et al., Selective delivery of low-affinity IL-2 to PD-1+ T cells rejuvenates antitumor immunity with reduced toxicity. J Clin Invest, 2022. 132(3).
13. Liao, S., et al., Targeting of LAK activity to CEA-expressing tumor cells with an anti-CEA scFv/IL-2 fusion protein. Anticancer Res, 2001. 21(3b): p. 1673-80.
14. Viitanen, R., et al., [(68)Ga]Ga-DOTA-Siglec-9 Detects Pharmacodynamic Changes of FAP-Targeted IL2 Variant Immunotherapy in B16-FAP Melanoma Mice. Front Immunol, 2022. 13: p. 901693.
15. Klein, C., et al., Cergutuzumab amunaleukin (CEA-IL2v), a CEA-targeted IL-2 variant-based immunocytokine for combination cancer immunotherapy: Overcoming limitations of aldesleukin and conventional IL-2-based immunocytokines. Oncoimmunology, 2017. 6(3): p. e1277306.

### Sequence Listing

wild-type IL-2 (SEQ ID NO:1)
Fc single chain of human IgG (SEQ ID NO:2) 
DKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
No-ADCC-mutated Fc region of IgG (SEQ ID NO:3) 
KTHTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALGAPIEKTISK
AKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK
knob-mutated Fc region of IgG (SEQ ID NO:4) 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALGAPIEKTIS
KAKGQPREPQVYTLPPCRDELTKNQVSLWCLVKGFYPSDIAVEWESNGQPENNYKTTP
PVLDSDGSFFLYSALTVDKSRWQQGNVFSCSVMHEALHNHYTQKSLSLSPGK
hole-mutated Fc region of IgG (SEQ ID NO:5) 
DKTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALGAPIEKTIS
KAKGQPREPQVCTLPPSRDELTKNQVSLSCAVKGFYPSDIAVEWESNGQPENNYKTTPP
VLDSDGSFKLVSKLTVDKSRWQQGNVFSCSVMHEALHNHYTQKSLSLSPGK
G4S linking sequence (SEQ ID NO:6)
   GGGGS
VL-CL of anti-TIM3 antibody (SEQ ID NO:7) 
AIQLTQSPSSLSASVGDRVTITCRASESVEYYGTSLMQWYQQKPGKAPKLLIYAASNVES
GVPSRFSGSGSGTDFTLTISSLQPEDFATYFCQQSRKDPSTFGGGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
VH-CH1 of anti-TIM3 antibody (SEQ ID NO:8) 
QVQLVQSGAEVKKPGSSVKVSCKASGYTFTSYNMHWVRQAPGQGLEWMGDIYPGNG
DTSYNQKFKGRVTITADKSTSTVYMELSSLRSEDTAVYYCARVGGAFPMDYWGQGTT
VTVSSASTKGPSVFPLAPSSKSTSGGTAALGCLVKDYFPEPVTVSWNSGALTSGVHTFPA
 VLQSSGLYSLSSVVTVPSSSLGTQTYICNVNHKPSNTKVDKKVEPKSC
TIM3 single chain variable fragment (ScFv) (SEQ ID NO:9) 
AIQLTQSPSSLSASVGDRVTITCRASESVEYYGTSLMQWYQQKPGKAPKLLIYAASNVES
GVPSRFSGSGSGTDFTLTISSLQPEDFATYFCQQSRKDPSTFGGGTKVEIKGGGGSGGGG
SGGGGSQVQLVQSGAEVKKPGSSVKVSCKASGYTFTSYNMHWVRQAPGQGLEWMGDI
YPGNGDTSYNQKFKGRVTITADKSTSTVYMELSSLRSEDTAVYYCARVGGAFPMDYW
GQGTTVTVSS
laIL2-Fc(hole) (SEQ ID NO:10) 
APTSSSTKKTQLQLEHLLLDLQMILNGINNYKNPKLTRMLTFKFYMPKKATELKHLQCL
EEELKPLEEVLNLAQSKNFHLRPRDLISNINVIVLELKGSETTFMCEYADETATIVEFLNR
WITFCQSIISTLTGGGGSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALGAPIEKTISKAKGQPREPQVCTLPPSRDELTKNQVSLSCAVKGFYPSDIAV
EWESNGQPENNYKTTPPVLDSDGSFKLVSKLTVDKSRWQQGNVFSCSVMHEALHNHY
TQKSLSLSPGK
VH-CH1-Fc (knob) of anti-TIM3 antibody (SEQ ID NO:11) 
QVQLVQSGAEVKKPGSSVKVSCKASGYTFTSYNMHWVRQAPGQGLEWMGDIYPGNG
DTSYNQKFKGRVTITADKSTSTVYMELSSLRSEDTAVYYCARVGGAFPMDYWGQGTT
VTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAP
EAAGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALGAPIEKTISKAKGQPREPQV
YTLPPCRDELTKNQVSLWCLVKGFYPSDIAVEWESNGQPENNYKTTPPVLDSDGSFFLY
SALTVDKSRWQQGNVFSCSVMHEALHNHYTQKSLSLSPGK
aTIM3 ScFv-Fc(knob) (SEQ ID NO:12) 
AIQLTQSPSSLSASVGDRVTITCRASESVEYYGTSLMQWYQQKPGKAPKLLIYAASNVES
GVPSRFSGSGSGTDFTLTISSLQPEDFATYFCQQSRKDPSTFGGGTKVEIKGGGGSGGGG
SGGGGSQVQLVQSGAEVKKPGSSVKVSCKASGYTFTSYNMHWVRQAPGQGLEWMGDI
YPGNGDTSYNQKFKGRVTITADKSTSTVYMELSSLRSEDTAVYYCARVGGAFPMDYW
GQGTTVTVSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKCKVSNK
ALGAPIEKTISKAKGQPREPQVYTLPPCRDELTKNQVSLWCLVKGFYPSDIAVEWESNG
QPENNYKTTPPVLDSDGSFFLYSALTVDKSRWQQGNVFSCSVMHEALHNHYTQKSLSL
 SPGK
VL-CL of anti-LAG3 antibody (SEQ ID NO: 13) 
EIVLTQSPGTLSLSPGERATLSCRASQSVSSYLAWYQQKPGQAPRLLIYGISSRATGIPDR
FSGSGSGTDFTLTISSLQPEDFATYYCQQANNFPLTFGGGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC
VH-CH1 of anti-LAG3 antibody (SEQ ID NO: 14) 
QVQLVQSGAEVKKPGSSVKVSCKASGGTFSSYAISWVRQAPGQGLEWMGGIIPIFGTAN
YAQKFQGRVTITADESTSTAYMELSSLRSEDTAVYYCAASTLRVPNPPYWGQGTLVTVS
SASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSC
anti-LAG3 scFv (SEQ ID NO:15) 
EIVLTQSPGTLSLSPGERATLSCRASQSVSSYLAWYQQKPGQAPRLLIYGISSRATGIPDR
FSGSGSGTDFTLTISSLQPEDFATYYCQQANNFPLTFGGGTKLEIKGGGGSGGGGSGGGG
SQVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCAASTLRVPNPPYWGQGTLVT
VSS
aLAG3 VH-CH1-Fc(knob) (SEQ ID NO:16) 
QVQLVQSGAEVKKPGSSVKVSCKASGGTFSSYAISWVRQAPGQGLEWMGGIIPIFGTAN
YAQKFQGRVTITADESTSTAYMELSSLRSEDTAVYYCAASTLRVPNPPYWGQGTLVTVS
SASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPEAAG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALGAPIEKTISKAKGQPREPQVYTLPP
CRDELTKNQVSLWCLVKGFYPSDIAVEWESNGQPENNYKTTPPVLDSDGSFFLYSALTV
DKSRWQQGNVFSCSVMHEALHNHYTQKSLSLSPGK
aLAG3 ScFv-Fc(knob) (SEQ ID NO:17) 
EIVLTQSPGTLSLSPGERATLSCRASQSVSSYLAWYQQKPGQAPRLLIYGISSRATGIPDR
FSGSGSGTDFTLTISSLQPEDFATYYCQQANNFPLTFGGGTKLEIKGGGGSGGGGSGGGG
SQVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCAASTLRVPNPPYWGQGTLVT
VSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEK
 
TISKAKGQPREPQVYTLPPCRDELTKNQVSLWCLVKGFYPSDIAVEWESNGQPENNYKT
TPPVLDSDGSFFLYSALTVDKSRWQQGNVFSCSVMHEALHNHYTQKSLSLSPGK
TIM3-InFc-laIL2: aTIM3(ScFv)-InFc-laIL2 (SEQ ID NO:18) 
AIQLTQSPSSLSASVGDRVTITCRASESVEYYGTSLMQWYQQKPGKAPKLLIYAASNVES
GVPSRFSGSGSGTDFTLTISSLQPEDFATYFCQQSRKDPSTFGGGTKVEIKGGGGSGGGG
SGGGGSQVQLVQSGAEVKKPGSSVKVSCKASGYTFTSYNMHWVRQAPGQGLEWMGDI
YPGNGDTSYNQKFKGRVTITADKSTSTVYMELSSLRSEDTAVYYCARVGGAFPMDYW
GQGTTVTVSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKCKVSNK
ALGAPIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSLSLSP
GKGGGGSAPTSSSTKKTQLQLEHLLLDLQMILNGINNYKNPKLTLMLTAKFYMPKKAT
ELKHLQCLEEELKPLEEVLNLAQSKNFHLRPRDLISNINVIVLELKGSETTFMCEYADET
ATIVEFLNRWITFCQSIISTLT
laIL2-InFc-TIM3: laIL2-InFc-aTIM3(ScFv) (SEQ ID NO:19) 
APTSSSTKKTQLQLEHLLLDLQMILNGINNYKNPKLTLMLTAKFYMPKKATELKHLQCL
EEELKPLEEVLNLAQSKNFHLRPRDLISNINVIVLELKGSETTFMCEYADETATIVEFLNR
WITFCQSIISTLTGGGGSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALGAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNHYT
QKSLSLSPGKGGGGSAIQLTQSPSSLSASVGDRVTITCRASESVEYYGTSLMQWYQQKP
GKAPKLLIYAASNVESGVPSRFSGSGSGTDFTLTISSLQPEDFATYFCQQSRKDPSTFGGG
TKVEIKGGGGSGGGGSGGGGSQVQLVQSGAEVKKPGSSVKVSCKASGYTFTSYNMHW
VRQAPGQGLEWMGDIYPGNGDTSYNQKFKGRVTITADKSTSTVYMELSSLRSEDTAVY
YCARVGGAFPMDYWGQGTTVTVSS
TIM3-laIL2-InFc: aTIM3(ScFv)-laIL2-InFc (SEQ ID NO:20) 
AIQLTQSPSSLSASVGDRVTITCRASESVEYYGTSLMQWYQQKPGKAPKLLIYAASNVES
GVPSRFSGSGSGTDFTLTISSLQPEDFATYFCQQSRKDPSTFGGGTKVEIKGGGGSGGGG
SGGGGSQVQLVQSGAEVKKPGSSVKVSCKASGYTFTSYNMHWVRQAPGQGLEWMGDI
YPGNGDTSYNQKFKGRVTITADKSTSTVYMELSSLRSEDTAVYYCARVGGAFPMDYW
GQGTTVTVSSGGGGSAPTSSSTKKTQLQLEHLLLDLQMILNGINNYKNPKLTLMLTAKF
YMPKKATELKHLQCLEEELKPLEEVLNLAQSKNFHLRPRDLISNINVIVLELKGSETTFM
 
CEYADETATIVEFLNRWITFCQSIISTLTGGGGSDKTHTCPPCPAPEAAGGPSVFLFPPKPK
DTLMISRTPEVTCVVVDVSHEDPEVKFNWYVDGVEVHNAKTKPREEQYNSTYRVVSVL
TVLHQDWLNGKEYKCKVSNKALGAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFS
CSVMHEALHNHYTQKSLSLSPGK
laIL2-TIM3-InFc: laIL2-aTIM3(ScFv)-Fc (SEQ ID NO:21) 
APTSSSTKKTQLQLEHLLLDLQMILNGINNYKNPKLTLMLTAKFYMPKKATELKHLQCL
EEELKPLEEVLNLAQSKNFHLRPRDLISNINVIVLELKGSETTFMCEYADETATIVEFLNR
WITFCQSIISTLTGGGGSAIQLTQSPSSLSASVGDRVTITCRASESVEYYGTSLMQWYQQK
PGKAPKLLIYAASNVESGVPSRFSGSGSGTDFTLTISSLQPEDFATYFCQQSRKDPSTFGG
GTKVEIKGGGGSGGGGSGGGGSQVQLVQSGAEVKKPGSSVKVSCKASGYTFTSYNMH
WVRQAPGQGLEWMGDIYPGNGDTSYNQKFKGRVTITADKSTSTVYMELSSLRSEDTAV
YYCARVGGAFPMDYWGQGTTVTVSSDKTHTCPPCPAPEAAGGPSVFLFPPKPKDTLMIS
RTPEVTCVVVDVSHEDPEVKFNWYVDGVEVHNAKTKPREEQYNSTYRVVSVLTVLHQ
DWLNGKEYKCKVSNKALGAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVM
HEALHNHYTQKSLSLSPGK
LAG3-InFc-laIL2: aLAG3(ScFv)-InFc-laIL2 (SEQ ID NO:22) 
EIVLTQSPGTLSLSPGERATLSCRASQSVSSYLAWYQQKPGQAPRLLIYGISSRATGIPDR
FSGSGSGTDFTLTISSLQPEDFATYYCQQANNFPLTFGGGTKLEIKGGGGSGGGGSGGGG
SQVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCAASTLRVPNPPYWGQGTLVT
VSSDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALGAPIEK
TISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGKGGGGS
APTSSSTKKTQLQLEHLLLDLQMILNGINNYKNPKLTLMLTAKFYMPKKATELKHLQCL
EEELKPLEEVLNLAQSKNFHLRPRDLISNINVIVLELKGSETTFMCEYADETATIVEFLNR
WITFCQSIISTLT
laIL2-InFc-LAG3: laIL2-InFc-aLAG3(ScFv) (SEQ ID NO:23) 
APTSSSTKKTQLQLEHLLLDLQMILNGINNYKNPKLTLMLTAKFYMPKKATELKHLQCL
EEELKPLEEVLNLAQSKNFHLRPRDLISNINVIVLELKGSETTFMCEYADETATIVEFLNR
WITFCQSIISTLTGGGGSDKTHTCPPCPAPEAAGGPSVFLFPPKPKDTLMISRTPEVTCVV
 
VDVSHEDPEVKFNWYVDGVEVHNAKTKPREEQYNSTYRVVSVLTVLHQDWLNGKEY
KCKVSNKALGAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNHYT
QKSLSLSPGKGGGGSEIVLTQSPGTLSLSPGERATLSCRASQSVSSYLAWYQQKPGQAPR
LLIYGISSRATGIPDRFSGSGSGTDFTLTISSLQPEDFATYYCQQANNFPLTFGGGTKLEIK
GGGGSGGGGSGGGGSQVQLVQSGAEVKKPGSSVKVSCKASGGTFSSYAISWVRQAPGQ
GLEWMGGIIPIFGTANYAQKFQGRVTITADESTSTAYMELSSLRSEDTAVYYCAASTLRV
PNPPYWGQGTLVTVSS
LAG3-laIL2-InFc: aLAG3(ScFv)-laIL2-InFc (SEQ ID NO:24) 
EIVLTQSPGTLSLSPGERATLSCRASQSVSSYLAWYQQKPGQAPRLLIYGISSRATGIPDR
FSGSGSGTDFTLTISSLQPEDFATYYCQQANNFPLTFGGGTKLEIKGGGGSGGGGSGGGG
SQVQLVQSGAEVKKPGSSVKVSCKASGGTFSSYAISWVRQAPGQGLEWMGGIIPIFGTA
NYAQKFQGRVTITADESTSTAYMELSSLRSEDTAVYYCAASTLRVPNPPYWGQGTLVT
VSSGGGGSAPTSSSTKKTQLQLEHLLLDLQMILNGINNYKNPKLTLMLTAKFYMPKKAT
ELKHLQCLEEELKPLEEVLNLAQSKNFHLRPRDLISNINVIVLELKGSETTFMCEYADET
ATIVEFLNRWITFCQSIISTLTGGGGSDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQD
WLNGKEYKCKVSNKALGAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHE
ALHNHYTQKSLSLSPGK
laIL2-LAG3-InFc: laIL2-aLAG3(ScFv)-InFc (SEQ ID NO:25) 
APTSSSTKKTQLQLEHLLLDLQMILNGINNYKNPKLTLMLTAKFYMPKKATELKHLQCL
EEELKPLEEVLNLAQSKNFHLRPRDLISNINVIVLELKGSETTFMCEYADETATIVEFLNR
WITFCQSIISTLTGGGGSEIVLTQSPGTLSLSPGERATLSCRASQSVSSYLAWYQQKPGQA
PRLLIYGISSRATGIPDRFSGSGSGTDFTLTISSLQPEDFATYYCQQANNFPLTFGGGTKLE
IKGGGGSGGGGSGGGGSQVQLVQSGAEVKKPGSSVKVSCKASGGTFSSYAISWVRQAP
GQGLEWMGGIIPIFGTANYAQKFQGRVTITADESTSTAYMELSSLRSEDTAVYYCAAST
LRVPNPPYWGQGTLVTVSSDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQDWLNGK
EYKCKVSNKALGAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALHN
HYTQKSLSLSPGK
aTIM3-VL-CL (SEQ ID NO:26) 
DIQMTQSPASLSASLGETVTIQCRASEDIYSGLAWFQQKPGKSPQLLIYGASSLQDGVPS
RFSGSGSGTQYSLKISSMQTEDEGVYFCQQGLKYPPTFGSGTKLEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC
aTIM3-VH-CH1-Fc(hole) (SEQ ID NO:27) 
EVQLVESGGGLVQPGRSLKLSCAASGFTFSDFYMAWVRQAPKKGLEWVASISYEGSST
YYGDSVMGRFTISRDNAKSTLYLQMNSLRSEDTATYYCARQREANWEDWGQGVMVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALGAPIEKTISKAKGQPREPQVYT
LPPCRDELTKNQVSLWCLVKGFYPSDIAVEWESNGQPENNYKTTPPVLDSDGSFFLYSA
LTVDKSRWQQGNVFSCSVMHEALHNHYTQKSLSLSPGK
aPD1-ScFv-Fc(hole) (SEQ ID NO:28) 
SYELTQPPSASVNVGETVKITCSGDQLPKYFADWFHQRSDQTILQVIYDDNKRPSGIPERI
SGSSSGTTATLTIRDVRAEDEGDYYCFSGYVDSDSKLYVFGSGTQLTVLGGGGSGGGGS
GGGGSEVRLLESGGGLVKPEGSLKLSCVASGFTFSDYFMSWVRQAPGKGLEWVAHIYT
KSYNYATYYSGSVKGRFTISRDDSRSMVYLQMNNLRTEDTATYYCTRDGSGYPSLDFW
GQGTQVTVSSDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKCKVSNK
ALGAPIEKTISKAKGQPREPQVYTLPPCRDELTKNQVSLWCLVKGFYPSDIAVEWESNG
QPENNYKTTPPVLDSDGSFFLYSALTVDKSRWQQGNVFSCSVMHEALHNHYTQKSLSL
SPGK
ahPD1-ScFv-Fc(hole) (SEQ ID NO:29) 
EIVLTQSPATLSLSPGERATLSCRASQSVSSYLAWYQQKPGQAPRLLIYDASNRATGIPA
RFSGSGSGTDFTLTISSLEPEDFAVYYCQQSSNWPRTFGQGTKVEIKGGGGSGGGGSGG
GGSQVQLVESGGGVVQPGRSLRLDCKASGITFSNSGMHWVRQAPGKGLEWVAVIWYD
GSKRYYADSVKGRFTISRDNSKNTLFLQMNSLRAEDTAVYYCATNDDYWGQGTLVTV
SSDKTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALGAPIEKTI
SKAKGQPREPQVYTLPPCRDELTKNQVSLWCLVKGFYPSDIAVEWESNGQPENNYKTT
PPVLDSDGSFFLYSALTVDKSRWQQGNVFSCSVMHEALHNHYTQKSLSLSPGK
aTIM3-VH-CH1-Fc (SEQ ID NO:30) 
EVQLVESGGGLVQPGRSLKLSCAASGFTFSDFYMAWVRQAPKKGLEWVASISYEGSST
YYGDSVMGRFTISRDNAKSTLYLQMNSLRSEDTATYYCARQREANWEDWGQGVMVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALGAPIEKTISKAKGQPREPQVYT
LPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK
IL2-Fc(hole) (SEQ ID NO:3 1) 
APTSSSTKKTQLQLEHLLLDLQMILNGINNYKNPKLTRMLTFKFYMPKKATELKHLQCL
EEELKPLEEVLNLAQSKNFHLRPRDLISNINVIVLELKGSETTFMCEYADETATIVEFLNR
WITFCQSIISTLTGGGGSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALGAPIEKTISKAKGQPREPQVCTLPPSRDELTKNQVSLSCAVKGFYPSDIAV
EWESNGQPENNYKTTPPVLDSDGSFKLVSKLTVDKSRWQQGNVFSCSVMHEALHNHY
TQKSLSLSPGK
laIL2 (SEQ ID NO:32)

## Claims

1. A fusion protein comprising at least one of:
a) an interleukin-2 (IL2) polypeptide;
b) an antibody against T cell surface molecule; and
c) a Fc region of an immunoglobulin.

2. The fusion protein according to claim 1, wherein the T cell surface molecule includes, but is not limited to, TIM3, LAG3, PD1, ICOS, GITR, CD27, CD137, 2B4, OX40, 4-1BB, TIGIT, CTLA4, VISTA, and CD8.

3. The fusion protein according to claim 1, wherein the Fc region of an immunoglobulin is a Fc region of natural immunoglobulin or a Fc region of an immunoglobulin without the ADCC effect caused by a gene mutation; preferably, the Fc region of an immunoglobulin is a Fc region of human IgG.

4. The fusion protein according to any one of claims 1-3, wherein
the T cell surface molecule is TIM3 or LAG3,
the antibody against T cell surface molecule is an anti-TIM3 antibody or an anti-LAG3 antibody.

5. The fusion protein according to any one of claims 1-4, wherein the fusion protein is a heterodimer comprising:
(1) a first monomer comprising sequentially from the N-terminus:
1) a wild-type IL-2 protein with the sequence as set forth in SEQ ID NO. 1, or a mutant of the wild-type IL-2 protein; preferably, the mutant of the wild-type IL-2 protein comprises any one or any combinations of the following mutation sites: R38L, F42A, H16K, D20K, R38A, F42K and K43E;
2) an essential linking structure (a G4S linking sequence); preferably, the linking sequence is as as set forth in SEQ ID NO.6; and
3) a Fc region of IgG with the sequence as set forth in SEQ ID NO. 2, or a mutated Fc region of IgG without the ADCC effect and the sequence as set forth in SEQ ID NO. 3 (InFc), or a knob-mutated Fc region of with the sequence as set forth in SEQ ID NO. 4, or a hole-mutated Fc region of with the sequence as set forth in SEQ ID NO. 5; and
(2) a second monomer comprising:
1) a Fab region of an anti-TIM3 antibody consisting of VL-KCL of the light chain of the anti-TIM3 antibody with the sequence as set forth in SEQ ID NO.7 and VH&CH1 of the heavy chain of the anti-TIM3 antibody with the sequence as set forth in SEQ ID NO.8; or,
an anti-TIM3 single chain variable fragment (ScFv) with the sequence as set forth in SEQ ID NO. 9; and
2) a Fc region of IgG with the sequence as set forth in SEQ ID NO. 2, or a mutated Fc region of IgG without the ADCC effect and the sequence as set forth in SEQ ID NO. 3 (InFc), or a knob-mutated Fc region of with the sequence as set forth in SEQ ID NO. 4, or a hole-mutated Fc region of with the sequence as set forth in SEQ ID NO. 5.

6. The fusion protein according to any one of claims 1-5, wherein the heterodimer comprises:
a first monomer, which is a polypeptide with the sequence as set forth in SEQ ID NO. 10 (laIL2-Fc(hole)); and
a second monomer, which is:
(1) a Fab construct of an anti-TIM3 antibody consisting of VL-KCL of the light chain of the anti-TIM3 antibody with the sequence as set forth in SEQ ID NO.7 and VH-CH1-Fc (knob) of the anti-TIM3 antibody with the sequence as set forth in SEQ ID NO.11; or
(2) a polypeptide with the sequence as set forth in SEQ ID NO. 12 or SEQ ID NO. 22 (aTIM3-ScFv-Fc (knob)).

7. The fusion protein according to claim 5, wherein, the second monomer comprises:
1) a Fab region of an antibody consisting of VL-CL of the light chain of the anti-LAG3 antibody with the sequence as set forth in SEQ ID NO: 13 and VH-CH1 of the heavy chain of the anti-LAG3 antibody with the sequence as set forth in SEQ ID NO: 14, or 2) an anti-LAG3 single chain variable fragment (ScFv) with the sequence as set forth in SEQ ID NO. 15;
and
3) a Fc region of IgG with the sequence as set forth in SEQ ID NO. 2, or a No-ADCC-mutated Fc region (InFc) of IgG with the sequence as set forth in SEQ ID NO. 3, or a knob-mutated Fc region with the sequence as set forth in SEQ ID NO. 4, or a hole-mutated Fc region with the sequence as set forth in SEQ ID NO. 5.

8. The fusion protein according to claim 6, wherein the second monomer is:
(1) a Fab construct of an anti-LAG3 antibody consisting of a polypeptide with the sequence as set forth in SEQ ID NO. 16 (aLAG3 VH-CH1-Fc (knob)) and the light chain variable region of the anti-LAG3 antibody with the sequence as set forth in SEQ ID NO. 13; or
(2) a polypeptide with the sequence as set forth in SEQ ID NO. 17 or SEQ ID NO. 22 (aLAG3 ScFv-Fc (knob)).

9. The fusion protein according to any one of claims 1-4, wherein the fusion protein is a homodimer, and wherein the monomer of the homodimer is:
a monomer consisting of one molecule of interleukin-2 and one molecule of anti-TIM3 antibody (aTIM3) or Fab region of the anti-LAG3 antibody (aLAG3) linked by any manner, or,
a monomer consisting of one molecule of interleukin-2 and one molecule of anti-LAG3 antibody (aLAG3) or (ScFv) of the anti-LAG3 antibody (aLAG3) linked by any manner.

10. The fusion protein according to claim 9, wherein the monomer of the homodimer comprises sequentially from the N-terminus:
(1) a Fab region/ScFv of the anti-TIM3 antibody (aTIM3) or the anti-LAG3 antibody (aLAG3); wherein, the Fab region is a Fab region of a humanized antibody or a Fab region ofa fully human antibody, and the ScFv is a ScFv of a humanized antibody or a ScFv of a fully human antibody;
(2) a Fc region of an antibody, which is a fully human wild-type Fc region or a No-ADCC-mutated Fc region (InFc);
(3) an essential linking structure (for example, a G4S linking sequence); preferably, the linking sequence is as set forth in SEQ ID NO.6; and
(4) a wild-type IL-2 with the sequence as set forth in SEQ ID NO. 1, or a mutant of the wild-type IL-2; wherein the mutant comprises any one or any combinations of the following mutation sites: R38L, F42A, H16K, D20K, R38A, F42K and K43E.

11. The fusion protein according to claim 9, wherein the monomer of the homodimer is:
(1) a polypeptide as set forth in SEQ ID NO. 18 (TIM3-InFc-laIL2: aTIM3(ScFv)-InFc-laIL2), or
(2) a polypeptide as set forth in SEQ ID NO. 19 (laIL2-InFc-TIM3: laIL2-Fc-aTIM3(ScFv)), or
(3) a polypeptide as set forth in SEQ ID NO. 20 (TIM3-laIL2-InFc: aTIM3(ScFv)-Fc-laIL2), or
(4) a polypeptide as set forth in SEQ ID NO. 21 (laIL2-TIM3-InFc: laIL2-aTIM3(ScFv)-Fc), or
(5) a polypeptide as set forth in SEQ ID NO. 22 (LAG3-InFc-laIL2: aLAG3(ScFv)-InFc-laIL2), or
(6) a polypeptide as set forth in SEQ ID NO. 23 (laIL2-InFc-LAG3: laIL2-Fc-aLAG3(ScFv)), or
(7) a polypeptide as set forth in SEQ ID NO. 24 (LAG3-laIL2-InFc: aLAG3(ScFv)-Fc-laIL2), or
(8) a polypeptide as set forth in SEQ ID NO. 25 (laIL2-LAG3-InFc: laIL2-aLAG3(ScFv)-Fc).

12. Use of the fusion protein of any one of claims 1-11:
(1) in the manufacture of an anti-tumor drug;
(2) in the manufacture of an anti-tumor drug used in combination with an immune checkpoint inhibitor;
(3) in the manufacture of an anti-tumor drug for overcoming a tolerance to an immune checkpoint inhibitor;
(4) in the manufacture of an anti-tumor drug used in combination with an adoptive T cell transfer therapy; or
(5) in the manufacture of an anti-tumor drug for overcoming a non-responsiveness to an adoptive T cell transfer therapy,
preferably, the tumor is selected from lung cancer, colorectal cancer, gastric cancer, melanoma, renal cancer, lymphoma such as B-cell lymphoma, prostate cancer, thyroid cancer, breast cancer, pancreatic cancer, liver cancer, cervical cancer, ovarian cancer or esophageal cancer.

13. The use according to claim 12, wherein the immune checkpoint inhibitor is an anti-PD-L1 antagonist; preferably, the anti-PD-L1 antagonist is an anti-PD-L1 antibody.

14. The use according to claim 12, wherein the T cell is an anti-tumor T cell; more preferably, the T cell is an anti-tumor CAR T cell or a structural analogue thereof.

15. A medicament or pharmaceutical composition comprising the fusion protein of any one of claims 1-12.

16. A kit comprising the fusion protein of any one of claims 1-12, preferably further comprising an anti-tumor drug selected from one or more of: an anti-PD-L1 antibody, an anti-PD1 antibody, an anti-TIGIT antibody and a chemotherapeutic drug, preferably thechemotherapeutic drug such as a tyrosine kinase inhibitor, nitrogen mustard, adriamycin, a platinum-based drug or paclitaxel.

17. An mRNA or a DNA encoding a first monomer or a second monomer of the fusion protein of any one of claims 5-8, or encoding a monomer of the fusion protein of any one of claims 9-11.
